# EUROPEAN PATENT SPECIFICATION

(11) **EP 4 233 935 B1**
(45) Date of publication and mention of the grant of the patent: **29.04.2026**
(21) Application number: 22305201.0
(22) Date of filing: 23.02.2022
(51) Int. Cl.: A61M 5/00

(54) **HOLDING DEVICE FOR MEDICAL CONTAINERS WITH REINFORCING RIBS FOR INCREASING STIFFNESS AND REDUCING DEFLECTION**
HALTEVORRICHTUNG FÜR MEDIZINISCHE BEHÄLTER MIT VERSTÄRKUNGSRIPPEN ZUR ERHÖHUNG DER STEIFIGKEIT UND ZUR VERRINGERUNG DER DURCHBIEGUNG
DISPOSITIF DE SUPPORT POUR RÉCIPIENTS MÉDICAUX AVEC DES NERVURES DE RENFORCEMENT POUR AUGMENTER LA RIGIDITÉ ET RÉDUIRE LA DÉFLEXION

(43) Date of publication of application: 30.08.2023
(60) Divisional application: 26165666.4
(73) Proprietor: Becton Dickinson France, 38800 Le Pont-de-Claix (FR)
(72) Inventor: REMPFER, Simon, 38260 St Hilaire de la Cote (FR); PAULET, Mathilde, 38100 Grenoble (FR); VAXELAIRE, Jérémie, 38000 Grenoble (FR); FLORES, Mario, 14080 TLALPAN (MX); EYMERY, Anaïs, 38450 Saint-Georges-de-Commiers (FR)
(74) Representative: Germain Maureau

(56) References cited:
- WO-A1-2014/130349
- DE-A1- 102020 203 992
- US-A1- 2014 014 654
- US-A1- 2020 156 840

## Description

### BACKGROUND OF THE INVENTION

### Field of the Invention

The present disclosure relates generally to holding devices for holding medical containers, such as cartridges, barrels, prefilled-syringes, stoppers, and/or pre-fillable syringes, in an upright position and, in particular, to holding devices including supporting structures, such as plates or trays, configured to be attached to containers or tubs for containing the medical containers.

### Description of Related Art

Medical containers, such as cartridges, barrels, prefilled-syringes, stoppers, and/or pre-fillable syringes, often need to be transported from one site to another site during or after manufacturing. For example, medical containers may be manufactured at a first site and then transported to a second site, where the medical containers are filled with a medical fluid. As used herein, a "medical fluid" can refer to a medication or another therapeutic agent used for treatment of chronic or acute conditions, as are known in the art. Exemplary therapeutic agents can include, for example, drugs, chemicals, biological, or biochemical substances that, when delivered in a therapeutically effective amount to the patient, achieve a desired therapeutic effect. In other examples, the medical containers can be manufactured and filled at a same first site and then transported to a second site for storage.

During transport, the medical containers can be held by a holding device, such as a tray, plate, and/or nest. A nest can be a plate-shaped tray configured to support multiple medical containers in an upright orientation. Nests can comprise multiple through-holes or openings aligned according to predetermined rows, with each through-hole being configured to receive one medical container. When inserted into the through-hole, the medical container is held in the upright orientation in a direction substantially orthogonal to the nest. The nest is usually placed inside a box-shaped tub with an open top, which can be sealed by a sealing cover. Removal of the medical containers from this tub can require peeling off the sealing cover, removing the nest holding the medical containers from the tub, and removing the medical containers from the nest by axially sliding the medical containers relative to the nest. Exemplary nests and tubs configured to contain medical containers are disclosed, for example, in: U.S. Patent No. 10,023,358 ("the '358 patent"), entitled "Packaging for containers;" U.S. Patent No. 10, 143,793 ("the '793 patent"), entitled "Tray for positioning elongated objects, in particular syringe bodies or syringes;" and U.S. Patent Appl. Pub. No. 2021/0236715A1 ("the '715 publication"), entitled "Holding Device Configured to Support a Plurality of Medical Containers Such as Syringes".

In many cases, the medical containers are filled with the medical fluid using an automated filling machine before the medical containers are removed from the nest and individually packaged. The nests can deform during filling due to forces exerted on the medical containers and/or nest by the automated filing machine. For example, when handled by the filling machine, the nests may bend or warp due to the weight of multiple medical containers held by the nest. As a result of deformation of the nest or trays, medical containers supported by the deformed trays may not be retained in the upright orientation, which may lead to some inaccuracy during the filling process and/or during a stoppering process. Deformation of the nests or trays may also cause problems during conveying of the filled nests or trays to different locations.

For these reasons, there is a need for nest and tray designs with increased stiffness in order to reduce deflection of the nest or tray during transport and filling. US-2014/014654, US-2020/156840, DE-10.2020.203992 and WO-2014/130349 disclose prior art devices. Avoiding deformation can improve accuracy of the filling process by avoiding misalignment between the medical containers contained by the tray or nest and the automated filling machines. The holding devices of the present disclosure are intended to provide such increased stiffness and to resist deflection or deformation under weight of multiple medical containers contained by the tray or nest.

### SUMMARY OF THE INVENTION

The invention is defined by the appended claims. According to an aspect of the disclosure, a holding device configured to support medical containers includes a supporting plate having an upper surface, a lower surface, and a plurality of openings through the supporting plate configured to receive the medical containers. The holding device also includes a plurality of chimneys protruding from the upper surface and/or the lower surface of the supporting plate at least partially enclosing the plurality of openings, such that the plurality of chimneys guide insertion of the medical containers into the plurality of openings; and a plurality of reinforcing ribs protruding from the upper surface and/or the lower surface of the supporting plate extending between the plurality of chimneys. The plurality of reinforcing ribs include a first end portion extending radially from a first chimney of the plurality of chimneys and a second end portion extending radially from an adjacent second chimney of the plurality of chimneys, such that the first end portion and the second end portion are offset from a virtual line extending between a center of the first chimney and a center of the adjacent second chimney.

According to another aspect of the disclosure, an assembly configured to support a plurality of medical containers includes a tub and the previously described holding device contained in the tub.

According to another aspect of the present disclosure, a method of manufacture for the previously described holding device includes injection molding or three-dimensional printing the support plate, the plurality of chimneys, and the plurality of reinforcing ribs of the holding device as a single part by a single injection molding or three-dimensional printing process.

### BRIEF DESCRIPTION OF THE DRAWINGS

FIG. 1A is a perspective view of a holding device for medical containers, according to an aspect of the present disclosure.
FIG. 1B is a top view of the holding device of FIG. 1A.
FIG. 1C is a schematic drawing showing an enlarged portion of a top view of the holding device of FIG. 1A.
FIG. 2A is a perspective view of another example of a holding device for medical containers, according to an aspect of the present disclosure.
FIG. 2B is a top view of the holding device of FIG. 2A.
FIG 3 is a top view of another example of a holding device, according to an aspect of the present disclosure.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the scope of the present invention.

For purposes of the description hereinafter, the terms "upper", "lower", "right", "left", "vertical", "horizontal", "top", "bottom", "lateral", "longitudinal", "transverse", and derivatives thereof shall relate to the invention as it is oriented in the drawing figures. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

With reference to the figures, the present disclosure is directed to holding devices 10, 10b, 10c for retaining multiple medical containers, such as multiple cartridges, barrels, prefilled-syringes, stoppers, and/or pre-fillable syringes, in an upright position. In particular, the holding devices 10, 10b, 10c can be configured to concurrently support multiple medical containers in the upright orientation during packaging, storing, transporting, and/or handling of the multiple medical containers with an automated filling machine.

A medical container, such as a syringe, is generally an elongate structure that comprises a hollow elongated barrel defining a reservoir for containing the medical fluid, a stopper located inside the barrel, and a plunger rod to move the stopper through the barrel to expel the medical fluid through a tip, fluid port, nozzle, or needle cannula at an end of the barrel. The reservoirs of the medical containers held by the holding devices 10, 10b, 10c of the present disclosure can contain less than about 50 mL, or preferably from about 1 mL to 10 mL, of the medical fluid. The medical containers can also include a flange at an end of the barrel, which can provide a surface for positioning a user's fingers (e.g., the index and the middle fingers), while the plunger rod is activated with the thumb. The holding device 10, 10b, 10c can be configured to retain fewer medical containers than most currently available trays or supporting plates. For example, the holding devices 10, 10b, 10c can be configured to contain 160 or fewer medical containers, preferably 100 or fewer medical containers, or more preferably, 50 or fewer medical containers, for example 42 medical containers.

In some examples, the holding devices 10, 10b, 10c can include protruding structures, such as ribs, ridges, angled surfaces, beads, channels, grooves, and similar protrusions, for increasing stiffness of the holding device 10, 10b, 10c and resisting deformation of the holding device 10, 10b, 10c. In particular, the holding devices 10, 10b, 10c can be configured to resist deformation when the holding devices 10, 10b, 10c are used with the automated filing machine, such that downward deflection of the holding device 10, 10b, 10c is 2.5 mm or less when the holding device 10, 10b, 10c is substantially fully loaded with filed medical containers. Specifically, the holdings devices 10, 10b, 10c can be configured to deflect by 2.5 mm or less, for a holding device 10, 10b, 10c having a thickness of 1.27 mm comprising 42 cavities loaded with filled 10 mL medical containers, as determined by finite element analysis (FEA). By contrast, a conventional holding device or plate with a plate thickness of 1.27 mm and including 42 cavities, but without structures for increasing stiffness, was found by FEA to deflect by over 20 mm.

As previously described, the holding devices 10, 10b, 10c of the present disclosure are configured to be disposed in a container, such as a tub. Exemplary tubs are described in the '358 patent, the '793 patent, and the '715 publication referred to previously. For example, the holding devices 10, 10b, 10c of the present disclosure can be disposed across the open top of a tub to cover an interior of the tub. The medical containers can be inserted through through-holes or openings of the holding devices 10, 10b, 10c, such that distal portions of the medical containers (i.e., portions of the medical containers that are farthest away from the portion of the device or container grasped or manipulated by a user) are in the interior of the tub and a sidewall of the medical container is supported by the through-hole or opening of the holding devices 10, 10b, 10c.

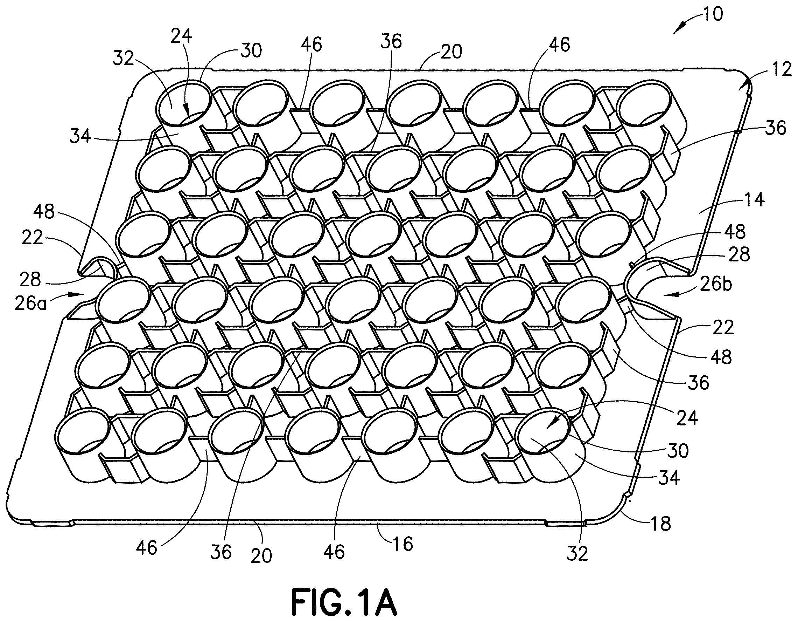

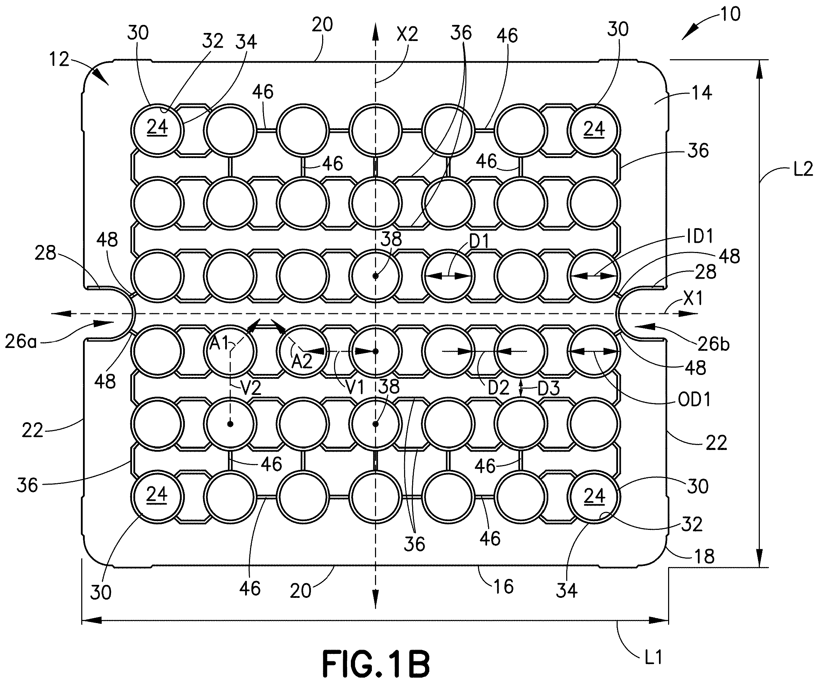

FIGS. 1A-1C illustrate an exemplary holding device 10 configured to support medical containers including features of the present disclosure. As shown in FIGS. 1A and 1B, the holding device 10 comprises a supporting plate 12 having a first or upper surface 14, a second or lower surface 16, and a peripheral edge 18 extending about the supporting plate 12 between the upper surface 14 and the lower surface 16. In some examples, the supporting plate 12 is substantially rectangular having longitudinal sides 20 and transverse sides 22. As used herein, the "longitudinal sides" refer to longer sides 20 of the supporting plate 12 having a length L1 (shown in FIG. 1B) and the "transverse sides" refer to shorter sides 22 of the supporting plate 12 having a length L2 (shown in FIG. 1B), which is shorter than the length L1. The supporting plate 12 may also be square, in which case the longitudinal sides 20 and the transverse sides 22 are the same length (i.e., L1 is equal to L2). As shown in FIG. 1B, the substantially rectangular supporting plate 12 defines a first axis X1 (also referred to as a longitudinal, horizontal, or x-axis) extending parallel to the longitudinal sides 20 of the supporting plate 12 and a second axis X2 (also referred to as a transverse, latitudinal, vertical, or y-axis) extending parallel to the transverse sides 22 of the supporting plate 12.

Dimensions of the supporting plate 12 will be determined by those skilled in the art based on the number of medical containers that can be supported by the holding device 10, dimensions of the medical containers, and/or dimensions of the tub or container to which the holding device 10 is mounted. For example, the length L1 can be from 228.8 mm to 231.2 mm and the length L2 can be from 197.1 mm to 199.5 mm. A thickness of the supporting plate can be from 1.0 mm to 2.0 mm or, preferably from about 1.3 mm to about 1.7 mm. In some examples, dimensions of the supporting plate 12 can be determined based on published standards for pre-filled syringe packaging dimensions (e.g., ISO 11040-7:2015(E): Packaging systems for sterilized sub-assembled syringes ready for filling).

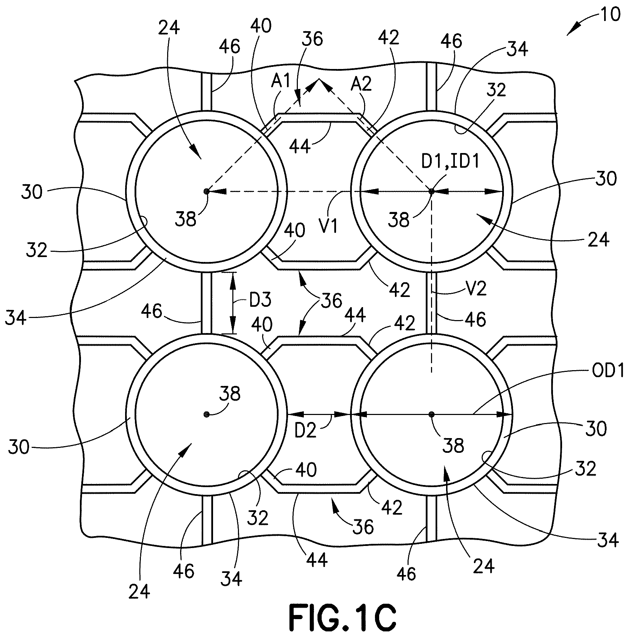

The supporting plate 12 further comprises multiple openings 24 through the supporting plate 12 configured to receive the medical containers. The openings 24 are sized to support to medical containers in an upright position. For example, the openings 24 can be wide enough so that a sidewall of the medical container passes through the openings 24, while preventing a flange or another protruding portion of the medical container from passing through the openings 24. Accordingly, when the medical container is inserted through one of the openings 24, the flange of the medical container may rest against the upper surface 14 of the supporting plate 12, thereby holding the medical container in the upright position. The dimensions of the openings 24 are generally dependent upon a size of the medical container intended to be used with the holding device 10. For example, an inner diameter D1 (shown in FIGS. 1B and 1C) of the openings 24 can be from about 18.06 mm to about 19.06 mm for a supporting plate 12 comprising 42 cavities. For a supporting plate 12 comprising 100 cavities, the inner diameter of the openings 24 can be from about 12.1 mm to about 12.35 mm.

In some examples, the supporting plate 12 can include one-hundred or fewer openings 24 or, preferably, fifty or fewer openings 24. The openings 24 can be arranged on the supporting plate 12 as rows and columns. For example, the supporting plate 12 can include at least five rows and at least five columns. In order for the supporting plate 12 to have a rectangular shape, in some examples, the supporting plate 12 includes a greater number of columns than rows. As shown in FIGS. 1A and 1B, in one example, the supporting plate 12 includes seven columns, which extend parallel to the transverse or second axis X2 and six rows, which extend parallel to the longitudinal or first axis X1 of the supporting plate 12. The openings 24 of each row and the openings 24 of each column can be equidistantly spaced. For example, an opening 24 of a row can be spaced apart from an adjacent opening 24 of the same row by a distance D2 (shown in FIGS. 1B and 1C) of from about 8.9 mm to about 11.11 mm. Similarly, an opening 24 of a column can be spaced apart from an adjacent opening 24 of the same column by a distance D3 (shown in FIGS. 1B and 1C) of about 8.76 mm to about 11.26 mm.

In some examples, openings 24 of the rows and columns are aligned. For example, openings 24 of adjacent rows can be aligned along virtual lines V2 extending parallel to the transverse or second axis X2. In particular, for a supporting plate 12 with seven columns and six rows, as shown in FIGS. 1A and 1B, each row includes seven openings 24, one opening 24 in each column. In other examples, openings 24 of the rows and/or columns can be offset. For example, each row can include four openings 24. However, the rows can be offset from adjacent rows, such that the supporting plate 12 includes seven columns.

The supporting plate 12 can also include cutouts 26a, 26b extending inwardly from other portions of the peripheral edge 18. For example, as shown in FIGS. 1A and 1B, the supporting plate 12 includes a first cutout 26a on one of the transverse sides 22 of the supporting plate 12 and a second cutout 26b on a second transverse side 22 of the supporting plate 12. The first and second cutouts 26a, 26b can be aligned along the longitudinal or first axis X1 of the supporting plate 12. In some examples, the cutouts 26a, 26b are a u-shaped gap or opening extending inwardly from the peripheral edge 18 of the supporting plate 12. The holding device 10 can also include a curved wall 28 extending from the upper surface 14 and/or the lower surface 16 of the supporting plate 12 and about at least a portion of the first cutout 26a and/or the second cutout 26b.

The holding device 10 further comprises tubular walls, members, or ridges (referred to herein as chimneys 30) protruding from the upper surface 14 and/or the lower surface 16 of the supporting plate 12. Each chimney 30 encloses or partially encloses one of the openings 24 and is positioned to guide insertion of the medical containers into the opening 24. The chimneys 30 can include a tubular wall comprising a cylindrical inner surface 32 having a diameter ID1 (shown in FIGS. 1B and 1C) substantially the same as the diameter D1 of the opening 24 enclosed or partially enclosed by the chimney 30 and a cylindrical outer surface 34 opposite the inner surface 32 having an outer diameter OD1. For example, for the supporting plate 12 comprising 42 chimneys 30, the inner diameter ID1 can be about 18.06 mm to about 19.06 mm and the outer diameter OD1 can be about 18.96 mm to about 21.06 mm, meaning that a thickness of the chimney 30 is about 0.9 mm to about 2.0 mm. A height of the chimneys 30 (e.g., a distance between the upper surface 14 of the supporting plate 12 and a top of the chimney 30) can be about 17.23 mm to about 17.73 mm.

As shown in FIGS. 1A and 1B, the chimneys 30 are tubular structures including the cylindrical inner surface 32 and the cylindrical outer surface 34. In other examples, the chimneys 30 can comprise axial slits or gaps extending through the chimneys 30, which separate the tubular wall of the chimney 30 into distinct segments or tabs. For example, a chimney 30 can include two axial slits (not shown), which separate the tubular wall into arcuate guide segments or guide tabs, similar to the slits and guide tabs shown in the '715 publication referred to previously.

The holding device 10 further comprises reinforcing ribs 36 protruding from the upper surface 14 and/or the lower surface 16 of the supporting plate 12 and extending between the chimneys 30. The reinforcing ribs 36 can be positioned to increase the stiffness of the holding device 10 and, in particular, to ensure that downward deflection of the holding device 10, when filled with medical containers, is 2.5 mm or less. A height of the reinforcing ribs (e.g., a vertical distance between the upper surface 14 of the supporting plate and a top of the reinforcing rib 36) can be slightly less than the height of the chimneys 30. For example, the height of the reinforcing ribs 36 can be about 2.0 mm to about 14.5 mm, which can be about 10% to about 83% of the height of the chimneys 30.

The reinforcing ribs 36 are angled or curved, when viewed from a top view, as shown in FIGS. 1B and 1C. Further, the reinforcing ribs 36 can be offset or spaced apart from a virtual line V1, V2 extending between a center 38 of a chimney 30 and a center 38 of an adjacent chimney 30. As shown in FIGS. 1B and 1C, the virtual lines V1, V2 are parallel to the axes X1, X2 of the supporting plate 12. The present inventors have determined that including angled or curved reinforcing ribs 36 increases stiffness of the supporting plate 12 compared to conventional plates 12 without ribs or which include straight ribs. Further, the reinforcing ribs 36 are unbranched structures, which do not intersect or directly connect to other reinforcing ribs 36.

More specifically, in some examples, as shown in FIG. 1C, the reinforcing ribs 36 are angled including a first end segment or portion 40 extending radially from a first chimney 30 in a first direction (shown by arrow A1 in FIG. 1C) and a second end segment or portion 42 extending radially from an adjacent second chimney 30 in a second direction (shown by arrow A2). The first end segment or portion 40 and the second end segment or portion 42 are offset from the virtual line V1 (shown in FIG. 1C) that extends between the center 38 of the first chimney 30 and the center 38 of the adjacent second chimney 30. The reinforcing ribs 36 further comprise an intermediate segment or portion 44 between the first end segment or portion 40 and the second end segment or portion 42 of the reinforcing rib 36 that is not radially aligned with either the first chimney 30 or the adjacent second chimney 30.

In some examples, as shown in FIGS. 1A and 1B, the holding device 10 includes both angled or curved reinforcing ribs 36 (also referred to herein as primary reinforcing ribs) and secondary straight reinforcing ribs 46. The primary reinforcing ribs 36 can extend longitudinally (e.g., parallel to the longitudinal or first axis X1 of the supporting plate 12) between a chimney 30 and an adjacent chimney 30 of a same row. In some examples, there can be two primary reinforcing ribs 36 extending between a chimney 30 and an adjacent chimney 30 of the same row. Further, the two primary reinforcing ribs 36 between the chimney 30 and the adjacent chimney 30 can be symmetrical about the virtual line V1 extending between the center 38 of the first chimney 30 and the center 38 of the adjacent second chimney 30, such that one of the reinforcing ribs 36 is angled toward a center of the supporting plate 12 and the other rib 36 is angled toward the peripheral edge 18 of the supporting plate 12.

In some examples, the secondary straight reinforcing ribs 46 can extend between outer chimneys 30 of the plurality of chimneys 30. As used herein, the "outer chimneys" refer to chimneys 30 that are proximate to the peripheral edge 18 of the supporting plate 12, namely the chimneys 30 of the rows closest to the longitudinal sides 20 of the supporting plate 12 and the chimneys 30 of the columns closest to the transverse sides 22 of the supporting plate 12. Straight reinforcing ribs 36 can also extend between a chimney 30 and an adjacent chimney 30 of a same column. As shown in FIG. 1C, the secondary straight reinforcing ribs 46 are co-extensive with the virtual lines V1, V2 that extend between the center 38 of the first chimney 30 and the center 38 of the adjacent second chimney 30. Further, the secondary straight ribs 46 are parallel to either the longitudinal or first axis X1 or the transverse or second axis X2 of the supporting plate 12.

As previously described, positioning for the primary and secondary reinforcing ribs 36, 46 of the holding device 10 can be designed and optimized to increase stiffness of the supporting plate 12. For example, some chimneys 30 can be connected to adjacent chimneys 30 by multiple primary and/or secondary reinforcing ribs 36, 46. Other chimneys 30 are connected to adjacent chimneys 30 by only a single primary reinforcing rib 36 or a single secondary reinforcing rib 46.

In the holding device 10 of FIGS. 1A-1C, , the chimneys 30 enclosing openings 24 of inner rows of the supporting plate 12 are connected together by two primary or angled reinforcing ribs 36 that are symmetrical about the virtual line V1 extending parallel to the longitudinal or first axis X1 of the supporting plate 12. Chimneys 30 enclosing many of the openings 24 of the outer rows are connected by the secondary straight reinforcing ribs 46 extending parallel to the longitudinal or first axis X1 of the supporting plate 12. Chimneys 30 enclosing openings 24 of inner columns are connected by the straight secondary ribs 46 extending parallel to the transverse or second axis X2. Chimneys 30 enclosing openings 24 of the outer columns are connected by a single angled primary reinforcing rib 36. The holding device 10 of FIGS. 1A and 1B also includes ribs 48 extending from several of the chimneys 30 to the walls 28 enclosing the cutouts 26a, 26b of the peripheral edge 18 of the supporting plate 12.

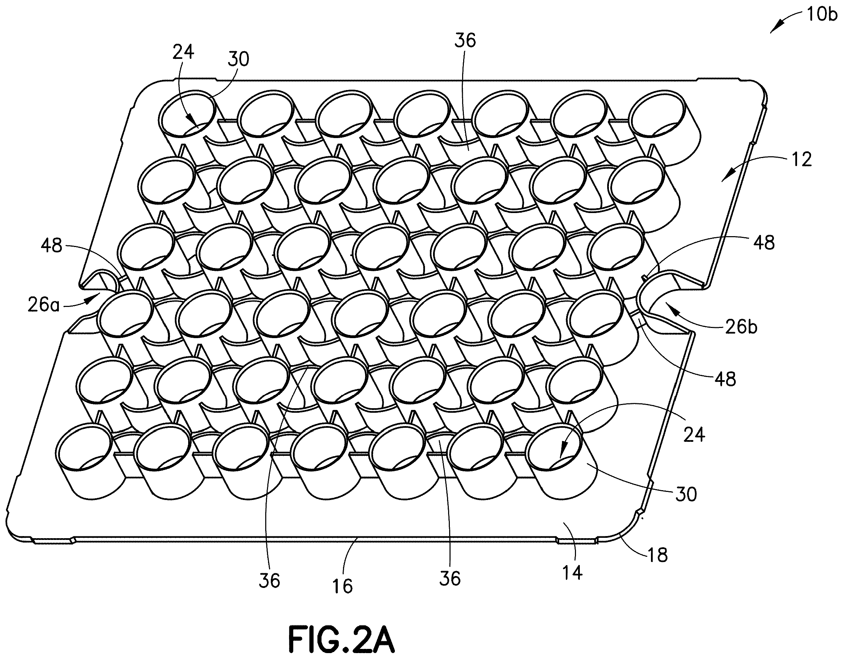

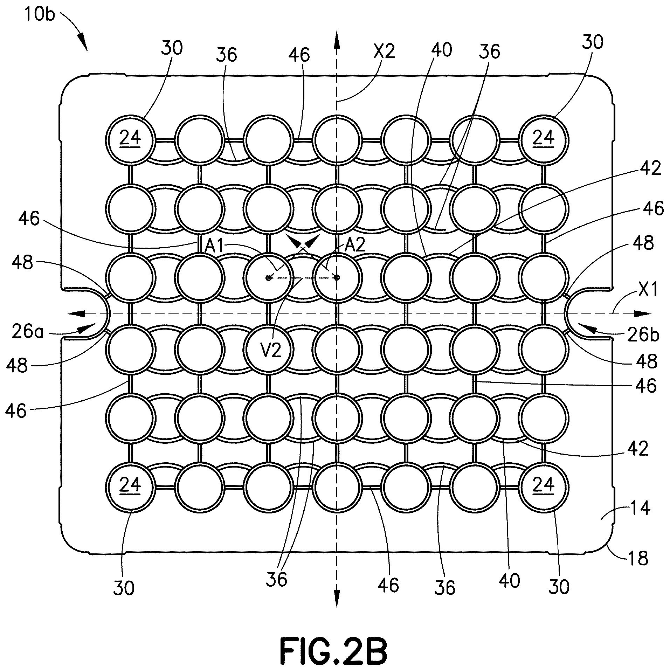

FIGS. 2A and 2B show another exemplary holding device 10b including a different arrangement of primary and secondary reinforcing ribs 36, 46. As shown in FIGS. 2A and 2B, the primary reinforcing ribs 36 include the first end segment or portion 40 extending radially from a first chimney 30 and the second end segment or portion 42 extending radially from an adjacent second chimney 30. The primary reinforcing ribs 36 are curved. As shown in FIGS. 2A and 2B, the chimneys 30 enclosing openings 24 of the inner rows of the supporting plate 12 are connected by two curved primary reinforcing ribs 36, which are symmetrical about the virtual line V1 parallel to the longitudinal or first axis X1 of the supporting plate 12. The chimneys 30 enclosing openings 24 of the outer rows are connected by both a curved primary reinforcing rib 36 and a straight secondary rib 46. Also, chimneys 30 enclosing openings 24 of the columns (e.g., both the inner columns and the outer columns) are connected by the secondary straight reinforcing ribs 46. The holding device 10b of FIGS. 2A and 2B also includes the ribs 48 extending from several of the chimneys 30 to the walls 28 enclosing the cutouts 26a, 26b of the peripheral edge 18 of the supporting plate 12.

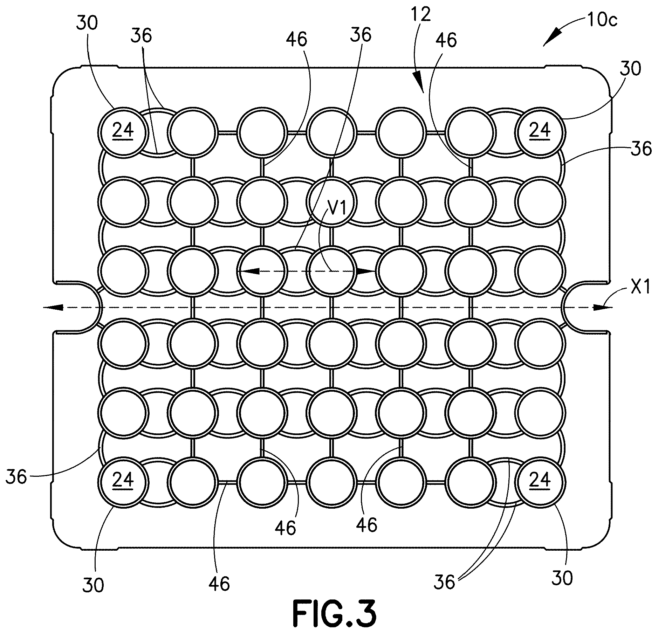

FIG. 3 shows another exemplary holding device 10c including a different arrangement of curved primary reinforcing ribs 36 and secondary straight reinforcing ribs 46. As shown in FIG. 3, the chimneys 30 enclosing openings 24 of the inner rows are connected by two curved primary reinforcing ribs 36, which are symmetrical about virtual lines V1 parallel to the longitudinal or first axis X1 of the supporting plate 12, similar to FIGS. 2A and 2B. Many of the chimneys 30 enclosing openings 24 of the outer rows are connected by straight secondary reinforcing ribs 46, though some of the chimneys 30 are also connected by two curved primary reinforcing ribs 36, as shown in FIG. 3. Chimneys 30 enclosing openings 24 of the inner columns are connected by straight secondary reinforcing ribs 46. Chimneys 30 enclosing openings 24 of the outer columns are connected by a single curved primary reinforcing rib 36.

The holding devices 10, 10b, 10c of the present disclosure can be made from various plastic materials commonly used for disposable medical packaging, containers, and/or devices. Further, the holding devices 10, 10b, 10c can be made by various forming and molding processes, as are known in the art. For example, the holding devices 10, 10b, 10c of the present disclosure can comprise a thermoplastic material, preferably polypropylene, or polyester, polycarbonate, polyethylene, polyethylene terephthalate, acrylonitrile butadiene styrene, or other injection moldable or formable resin materials, as are known in the art. In some examples, the supporting plate 12, chimneys 30, and primary and secondary reinforcing ribs 36, 46 can be integrally formed from a same material, such as from the same thermoplastic material. In some examples, the holding devices 10, 10b, 10c are formed by a single injection molding process, in which the holding device 10, 10b, 10c is formed by injecting a fluid polymer material between a first tool and a second tool in a single process. In order to optimize the holding devices 10, 10b, 10c for injection molding, in some examples, vertical or substantially vertical surfaces of the holding device 10, 10b, 10c, such as the inner surface 32 and/or the outer surface 34 of the chimneys 30, can include a slightly sloped surface having a draft angle (e.g., an angle of about 0.1 degree to 4.0 degrees, for example, 1.0 degree or 0.5 degree). Including substantially vertical surfaces with a slight slope or draft angle makes the molded part easier to remove from the mold following injection molding. In other examples, the holding devices 10, 10b, 10c can be made by three-dimensional printing by processes known in the art.

While examples of the holding devices 10, 10b, 10c are shown in the accompanying figures and described hereinabove in detail, other examples will be apparent to, and readily made by, those skilled in the art without departing from the scope of the invention. Accordingly, the foregoing description is intended to be illustrative rather than restrictive. The invention described hereinabove is defined by the appended claims.

## Claims

1. A holding device (10, 10b, 10c) configured to support medical containers, comprising:
a supporting plate (12) comprising an upper surface (14), a lower surface (16), and a plurality of openings (24) through the supporting plate (12) configured to receive the medical containers;
a plurality of chimneys (30) protruding from the upper surface (14) and/or the lower surface (16) of the supporting plate (12) at least partially enclosing the plurality of openings (24), such that the plurality of chimneys (30) guide insertion of the medical containers into the plurality of openings (24); and
a plurality of reinforcing ribs (36, 46) protruding from the upper surface (14) and/or the lower surface (16) of the supporting plate (12) extending between the plurality of chimneys (30),
wherein the plurality of reinforcing ribs (36, 46) comprise primary reinforcing ribs (36) comprising a first end portion (40) extending radially from a first chimney (30) of the plurality of chimneys (30) and a second end portion (42) extending radially from an adjacent second chimney (30) of the plurality of chimneys, such that the first end portion (40) and the second end portion (42) are offset from a virtual line (V1) extending between a center (38) of the first chimney (30) and a center (38) of the adjacent second chimney (30);
wherein the plurality of primary reinforcing ribs (36) further comprise an intermediate portion (44) between the first end portion (40) and the second end portion (42) that is radially aligned with neither the first end portion (40) nor the second end portion (42), and wherein the plurality of reinforcing ribs are not directly connected to any other of the plurality of reinforcing ribs.

2. The holding device (10, 10b, 10c) of claim 1, wherein the first end portion (40) extends from the first chimney (30) in a first direction and the second end portion (42) extends from the second chimney (30) in a second direction, which is different from the first direction.

3. The holding device (10, 10b, 10c) of claim 1, wherein the plurality of openings (34) are arranged on the supporting plate (12) as a plurality of rows and a plurality of columns.

4. The holding device (10, 10b, 10c) of claim 3, wherein the plurality of reinforcing ribs comprise primary reinforcing ribs (36) extending between the first chimney (30) and the adjacent second chimney (30) of a same row.

5. The holding device (10, 10b, 10c) of claim 4, wherein there are at least two primary reinforcing ribs (36) between the first chimney (30) and the adjacent second chimney (30) of the same row, and
wherein the at least two primary reinforcing ribs (36) between the first chimney (30) and the adjacent second chimney (30) are symmetrical about the virtual line (V1) extending between the center (38) of the first chimney (30) and the center (38) of the adjacent second chimney (30).

6. The holding device (10, 10b, 10c) of claim 4, further comprising a plurality of straight secondary ribs (46) extending between a first chimney (30) and a second chimney (30) of the same column.

7. The holding device (10, 10b, 10c) of claim 1, wherein the supporting plate (12) has a thickness of 1.27 mm and comprises 42 openings (24) configured to receive medical containers, and wherein, when fully loaded with filled 10mL medical containers, deflection of the supporting plate (12) is less than 2.5 mm in a vertical direction.

8. The holding device (10, 10b, 10c) of any preceding claim, wherein the supporting plate (12), plurality of chimneys (30), and plurality of reinforcing ribs (36, 46) comprise a thermoplastic polymer and/or are integrally formed by injection molding.

9. The holding device (10, 10b, 10c) of any preceding claim, wherein the supporting plate (12) further comprises a peripheral edge (18) extending between the upper surface (14) and the lower surface (16) of the supporting plate (12), and
wherein the peripheral edge (18) comprises at least one cutout (26a, 26b) extending inward from other portions of the peripheral edge.

10. The holding device (10, 10b, 10c) of claim 9, further comprising a curved wall (28) extending from the upper surface (14) and/or the lower surface (16) and about at least a portion of the at least one cutout (26a, 26b).

11. The holding device (10, 10b, 10c) of any preceding claim, wherein the supporting plate 12 comprises less than 100 openings, and/or wherein the plurality of chimneys (30) comprise a tubular wall comprising a cylindrical inner surface (32) having a diameter (ID1) substantially the same as the diameter (D1) of the opening (24) enclosed by the chimney (30) and a cylindrical outer surface (34) opposite the inner surface (32).

12. The holding device (10, 10b, 10c) of and preceding claim, wherein the plurality of openings (34) and the plurality of chimneys (30) are configured to receive barrels of syringes or cartridges containing a fluid volume of less than about 50 mL.

13. The holding device (10, 10b, 10c) of claim 1, wherein at least one of the plurality of primary reinforcing ribs (36) is curved or angled.

14. An assembly configured to support a plurality of medical containers, the assembly comprising:
a tub; and
the holding device (10, 10b, 10c) of any of claims 1 to 13 contained in the tub.

15. A method of manufacture for the holding device (10, 10b, 10c) of any of claims 1 to 13, the method comprising injection molding or three-dimensional printing the support plate (12), the plurality of chimneys (30), and the plurality of reinforcing ribs (36, 46) of the holding device as a single part by a single injection molding or three-dimensional printing process.

## Patentansprüche

1. Haltevorrichtung (10, 10b, 10c), die so eingerichtet ist, dass sie medizinische Behältern trägt, umfassend:
eine Stützplatte (12), die eine obere Oberfläche (14), eine untere Oberfläche (16) und eine Vielzahl von Öffnungen (24) durch die Stützplatte (12) umfasst, die so eingerichtet sind, dass sie die medizinischen Behälter aufnimmt;
eine Vielzahl von Zylindern (30), die aus der oberen Oberfläche (14) und/oder der unteren Oberfläche (16) der Stützplatte (12) hervorstehen und die die Vielzahl von Öffnungen (24) zumindest teilweise umschließen, so dass die Vielzahl von Zylindern (30) das Einführen der medizinischen Behälter in die Vielzahl von Öffnungen (24) führen; und
eine Vielzahl von Verstärkungsrippen (36, 46), die aus der oberen Oberfläche (14) und/oder der unteren Oberfläche (16) der Stützplatte (12) hervorstehen und sich zwischen der Vielzahl von Zylindern (30) erstrecken,
wobei die Vielzahl von Verstärkungsrippen (36, 46) primäre Verstärkungsrippen (36) umfassen, die einen ersten Endabschnitt (40), der sich radial von einem ersten Zylinder (30) der Vielzahl von Zylindern (30) erstreckt, und einen zweiten Endabschnitt (42) umfassen, der sich radial von einem benachbarten zweiten Zylinder (30) der Vielzahl von Zylindern erstreckt, so dass der erste Endabschnitt (40) und der zweite Endabschnitt (42) von einer virtuellen Linie (V1) versetzt sind, die sich zwischen einer Mitte (38) des ersten Zylinders (30) und einer Mitte (38) des benachbarten zweiten Zylinders (30) erstreckt;
wobei die Vielzahl von primären Verstärkungsrippen (36) ferner einen Zwischenabschnitt (44) zwischen dem ersten Endabschnitt (40) und dem zweiten Endabschnitt (42) umfassen, der radial weder mit dem ersten Endabschnitt (40) noch dem zweiten Endabschnitt (42) ausgerichtet ist, und wobei die Vielzahl von Verstärkungsrippen nicht direkt mit einer anderen der Vielzahl von Verstärkungsrippen verbunden sind.

2. Haltevorrichtung (10, 10b, 10c) nach Anspruch 1, wobei sich der erste Endabschnitt (40) vom ersten Zylinder (30) in eine erste Richtung erstreckt und der zweite Endabschnitt (42) vom zweiten Zylinder (30) in eine zweite Richtung erstreckt, die sich von der ersten Richtung unterscheidet.

3. Haltevorrichtung (10, 10b, 10c) nach Anspruch 1, wobei die Vielzahl von Öffnungen (34) auf der Stützplatte (12) als eine Vielzahl von Reihen und eine Vielzahl von Spalten angeordnet sind.

4. Haltevorrichtung (10, 10b, 10c) nach Anspruch 3, wobei die Vielzahl von Verstärkungsrippen primäre Verstärkungsrippen (36) umfassen, die sich zwischen dem ersten Zylinder (30) und dem benachbarten zweiten Zylinder (30) derselben Reihe erstrecken.

5. Haltevorrichtung (10, 10b, 10c) nach Anspruch 4, wobei sich mindestens zwei primäre Verstärkungsrippen (36) zwischen dem ersten Zylinder (30) und dem benachbarten zweiten Zylinder (30) derselben Reihe befinden, und
wobei die mindestens zwei primären Verstärkungsrippen (36) zwischen dem ersten Zylinder (30) und dem benachbarten zweiten Zylinder (30) symmetrisch um die virtuelle Linie (V1) sind, die sich zwischen der Mitte (38) des ersten Zylinders (30) und der Mitte (38) des benachbarten zweiten Zylinders (30) erstreckt.

6. Haltevorrichtung (10, 10b, 10c) nach Anspruch 4, ferner umfassend eine Vielzahl von geraden sekundären Rippen (46), die sich zwischen einem ersten Zylinder (30) und einem zweiten Zylinder (30) derselben Spalte erstrecken.

7. Haltevorrichtung (10, 10b, 10c) nach Anspruch 1, wobei die Stützplatte (12) eine Dicke von 1,27 mm aufweist und 42 Öffnungen (24) umfasst, die so eingerichtet sind, dass sie medizinische Behälter aufnehmen, und wobei die Durchbiegung der Stützplatte (12) bei vollständiger Beladung mit gefüllten medizinischen 10-ml-Behältern in vertikaler Richtung weniger als 2,5 mm beträgt.

8. Haltevorrichtung (10, 10b, 10c) nach einem der vorhergehenden Ansprüche, wobei die Stützplatte (12), eine Vielzahl von Zylindern (30) und eine Vielzahl von Verstärkungsrippen (36, 46) ein thermoplastisches Polymer umfassen und/oder integral durch Spritzguss geformt sind.

9. Haltevorrichtung (10, 10b, 10c) nach einem der vorhergehenden Ansprüche, wobei die Stützplatte (12) ferner eine umlaufende Kante (18) umfasst, die sich zwischen der oberen Oberfläche (14) und der unteren Oberfläche (16) der Stützplatte (12) erstreckt, und
wobei die umlaufende Kante (18) mindestens einen Ausschnitt (26a, 26b) umfasst, der sich von anderen Abschnitten der umlaufenden Kante nach innen erstreckt.

10. Haltevorrichtung (10, 10b, 10c) nach Anspruch 9, ferner umfassend eine gekrümmte Wand (28), die sich von der oberen Oberfläche (14) und/oder der unteren Oberfläche (16) und um etwa mindestens einen Abschnitt der mindestens einen Ausschnitt (26a, 26b) erstreckt.

11. Haltevorrichtung (10, 10b, 10c) nach einem der vorhergehenden Ansprüche, wobei die Stützplatte 12 weniger als 100 Öffnungen umfasst und/oder wobei die Vielzahl von Zylindern (30) eine rohrförmige Wand umfassen, die eine zylindrische Innenfläche (32) mit einem Durchmesser (ID1), der im Wesentlichen gleich dem Durchmesser (D1) der von dem Zylinder (30) umschlossenen Öffnung (24) ist, und eine der Innenfläche (32) gegenüberliegende zylindrische Außenfläche (34) umfasst.

12. Haltevorrichtung (10, 10b, 10c) nach einem der vorhergehenden Ansprüche, wobei die Vielzahl von Öffnungen (34) und die Vielzahl von Zylindern (30) so eingerichtet sind, dass sie Spritzenkörper oder Kartuschen aufnehmen, die ein Flüssigkeitsvolumen von weniger als etwa 50 ml enthalten.

13. Haltevorrichtung (10, 10b, 10c) nach Anspruch 1, wobei mindestens eine der Vielzahl von primären Verstärkungsrippen (36) gebogen oder abgewinkelt ist.

14. Baugruppe, die so eingerichtet ist, dass sie eine Vielzahl von medizinischen Behältern trägt, wobei die Baugruppe Folgendes umfasst:
eine Wanne; und
die in der Wanne enthaltene Haltevorrichtung (10, 10b, 10c) nach einem der Ansprüche 1 bis 13.

15. Herstellungsverfahren für die Haltevorrichtung (10, 10b, 10c) nach einem der Ansprüche 1 bis 13, wobei das Verfahren das Spritzgießen oder das dreidimensionale Drucken der Stützplatte (12), der Vielzahl von Zylindern (30) und der Vielzahl von Verstärkungsrippen (36, 46) der Haltevorrichtung als ein einziges Teil durch einen einzigen Spritzguss- oder dreidimensionalen Druckvorgang umfasst.

## Revendications

1. Dispositif de maintien (10, 10b, 10c) configuré pour supporter des récipients médicaux, comprenant :
une plaque de support (12) comprenant une surface supérieure (14), une surface inférieure (16) et une pluralité d'ouvertures (24) à travers la plaque de support (12) configurées pour recevoir les récipients médicaux ;
une pluralité de cheminées (30) faisant saillie de la surface supérieure (14) et/ou de la surface inférieure (16) de la plaque de support (12) et entourant au moins partiellement la pluralité d'ouvertures (24), de sorte que la pluralité de cheminées (30) guident l'insertion des récipients médicaux dans la pluralité d'ouvertures (24) ; et
une pluralité de nervures de renforcement (36, 46) faisant saillie de la surface supérieure (14) et/ou de la surface inférieure (16) de la plaque de support (12) s'étendant entre la pluralité de cheminées (30),
dans lequel la pluralité de nervures de renforcement (36, 46) comprend des nervures de renforcement primaires (36) comprenant une première partie d'extrémité (40) s'étendant radialement à partir d'une première cheminée (30) de la pluralité de cheminées (30) et une deuxième partie d'extrémité (42) s'étendant radialement à partir d'une deuxième cheminée adjacente (30) de la pluralité de cheminées, de sorte que la première partie d'extrémité (40) et la deuxième partie d'extrémité (42) soient décalées par rapport à une ligne virtuelle (V1) s'étendant entre un centre (38) de la première cheminée (30) et un centre (38) de la deuxième cheminée adjacente (30) ;
dans lequel la pluralité de nervures de renforcement primaires (36) comprennent en outre une partie intermédiaire (44) entre la première partie d'extrémité (40) et la deuxième partie d'extrémité (42) qui n'est alignée radialement ni avec la première partie d'extrémité (40) ni avec la deuxième partie d'extrémité (42), et dans lequel la pluralité de nervures de renforcement n'est pas directement reliée à une autre nervure de la pluralité de nervures de renforcement.

2. Dispositif de maintien (10, 10b, 10c) selon la revendication 1, dans lequel la première partie d'extrémité (40) s'étend à partir de la première cheminée (30) dans une première direction et la deuxième partie d'extrémité (42) s'étend à partir de la deuxième cheminée (30) dans une deuxième direction, qui est différente de la première direction.

3. Dispositif de maintien (10, 10b, 10c) selon la revendication 1, dans lequel la pluralité d'ouvertures (34) sont agencées sur la plaque de support (12) sous la forme d'une pluralité de rangées et d'une pluralité de colonnes.

4. Dispositif de maintien (10, 10b, 10c) selon la revendication 3, dans lequel la pluralité de nervures de renforcement comprend des nervures de renforcement primaires (36) s'étendant entre la première cheminée (30) et la deuxième cheminée adjacente (30) d'une même rangée.

5. Dispositif de maintien (10, 10b, 10c) selon la revendication 4, dans lequel il y a au moins deux nervures de renforcement primaires (36) entre la première cheminée (30) et la deuxième cheminée adjacente (30) de la même rangée, et
dans lequel les au moins deux nervures de renforcement primaires (36) entre la première cheminée (30) et la deuxième cheminée adjacente (30) sont symétriques par rapport à la ligne virtuelle (V1) s'étendant entre le centre (38) de la première cheminée (30) et le centre (38) de la deuxième cheminée adjacente (30).

6. Dispositif de maintien (10, 10b, 10c) selon la revendication 4, comprenant en outre une pluralité de nervures secondaires droites (46) s'étendant entre une première cheminée (30) et une deuxième cheminée adjacente (30) de la même colonne.

7. Dispositif de maintien (10, 10b, 10c) selon la revendication 1, dans lequel la plaque de support (12) a une épaisseur de 1,27 mm et comprend 42 ouvertures (24) configurées pour recevoir des récipients médicaux, et dans lequel, lorsqu'il est entièrement chargé de récipients médicaux de 10 ml remplis, la déflexion de la plaque de support (12) est inférieure à 2,5 mm dans une direction verticale.

8. Dispositif de maintien (10, 10b, 10c) selon l'une des revendications précédentes, dans lequel la plaque de support (12), la pluralité de cheminées (30) et la pluralité de nervures de renforcement (36, 46) comprennent un polymère thermoplastique et/ou sont formées d'un seul tenant par moulage par injection.

9. Dispositif de maintien (10, 10b, 10c) selon l'une des revendications précédentes, dans lequel la plaque de support (12) comprend en outre un bord périphérique (18) s'étendant entre la surface supérieure (14) et la surface inférieure (16) de la plaque de support (12), et
dans lequel le bord périphérique (18) comprend au moins une découpe (26a, 26b) s'étendant vers l'intérieur à partir d'autres parties du bord périphérique.

10. Dispositif de maintien (10, 10b, 10c) selon la revendication 9, comprenant en outre une paroi incurvée (28) s'étendant depuis la surface supérieure (14) et/ou la surface inférieure (16) et autour d'au moins une partie de l'au moins une découpe (26a, 26b).

11. Dispositif de maintien (10, 10b, 10c) selon l'une des revendications précédentes, dans lequel la plaque de support (12) comprend moins de 100 ouvertures, et/ou dans lequel la pluralité de cheminées (30) comprennent une paroi tubulaire comprenant une surface intérieure cylindrique (32) ayant un diamètre (ID1) sensiblement identique au diamètre (D1) de l'ouverture (24) entourée par la cheminée (30) et une surface extérieure cylindrique (34) opposée à la surface intérieure (32).

12. Dispositif de maintien (10, 10b, 10c) selon la revendication précédente, dans lequel la pluralité d'ouvertures (34) et la pluralité de cheminées (30) sont configurées pour recevoir des cylindres de seringues ou des cartouches contenant un volume de fluide inférieur à environ 50 ml.

13. Dispositif de maintien (10, 10b, 10c) selon la revendication 1, dans lequel au moins l'une de la pluralité de nervures de renforcement primaires (36) est incurvée ou inclinée.

14. Ensemble configuré pour supporter une pluralité de récipients médicaux, l'ensemble comprenant :
une cuve; et
le dispositif de maintien (10, 10b, 10c) selon l'une des revendications 1 à 13, contenu dans la cuve.

15. Procédé de fabrication du dispositif de maintien (10, 10b, 10c) selon l'une des revendications 1 à 13, le procédé comprenant le moulage par injection ou l'impression tridimensionnelle de la plaque de support (12), de la pluralité de cheminée (30) et de la pluralité de nervures de renforcement (36, 46) du dispositif de maintien en une seule pièce par un seul procédé de moulage par injection ou d'impression tridimensionnelle.
